# EUROPÄISCHE PATENTSCHRIFT

(11) **EP 1 450 890 B1**
(45) Veröffentlichungstag und Bekanntmachung des Hinweises auf die Patenterteilung: **07.12.2005**
(21) Anmeldenummer: 02779156.5
(22) Anmeldetag: 11.10.2002
(51) Int. Cl.: A61M 25/00

(54) **EINRICHTUNG ZUR AUFBEREITUNG VON BALLONKATHETERN, INSBESONDERE HERZKATHETERN**
DEVICE FOR PREPARING BALLOON CATHETERS, ESPECIALLY HEART CATHETERS
DISPOSITIF POUR PREPARER DES CATHETERS A BALLON, NOTAMMENT DES CATHETERS CARDIAQUES

(30) Priorität: 11.10.2001 DE 10150152; 07.03.2002 DE 10209993
(43) Veröffentlichungstag der Anmeldung: 01.09.2004
(73) Patentinhaber: Vanguard AG Medical Services for Europe, 10117 Berlin (DE)
(72) Erfinder: KRAFT, Marc, 15366 Dahlwitz-Hoppegarten (DE); GUTZMER, Thomas, 13599 Berlin (DE); BERNHARD, Ringo, 15745 Wildau (DE)
(74) Vertreter: von Puttkamer, Nikolaus
(86) Internationale Anmeldenummer: PCT/DE2002/003841
(87) Internationale Veröffentlichungsnummer: WO 2003/033032

(56) Entgegenhaltungen:
- EP-A- 0 890 337
- WO-A-97/32610
- DE-A- 4 317 601
- US-A- 4 380 530
- US-A- 5 310 524
- US-A- 5 871 692
- US-A- 6 036 928

## Beschreibung

Die vorliegende Erfindung betrifft eine Einrichtung zur Aufbereitung, insbesondere der Reinigung und Desinfektion, von Kathetern, insbesondere Herzkathetern, nach dem Oberbegriff des Patentanspruches 1 und ein Aufbereitungsverfahren.

Bekannte Ballonkatheter des sogenannten "Rapid-Exchange"-Typs, die beispielsweise verwendet werden, um Stenosen der Herzkranzgefäße aufzuweiten, bestehen üblicherweise aus einem proximalen Abschnitt und einem distalen Abschnitt. Mit dem proximalen Abschnitt ist ein Ballonanschlussteil verbunden, an dem eine Inflationsspritze angeschlossen werden kann. Durch diese wird ein Medium zur Aufweitung des Ballons eingeführt. An einem Ort vom Ballonanschlussteil entfernt beginnt bei diesem Kathetertyp ein zum distalen Ende hin im Ballonlumen verlaufendes Führungsdrahtlumen, wobei sich am Ort eine Drahtaustrittsstelle befindet. Beim Gebrauch des Katheters wird durch die Drahtaustrittstelle ein sogenannter Führungsdraht eingeschoben, der bei der Dilatation zunächst bis zur Stenose vorgeschoben wird und auf dem dann der Katheter mit seinem distalen Ende voran verschoben wird, bis sich der Ballon im Bereich der Stenose befindet.

Bei Ballonkathetern des "Over-the-wire"-Typs, die verwendet werden, um Stenosen peripherer Blutgefäße, der Herzkranzgefäße oder von Teilen beziehungsweise Gangsystemen des Gastrointestinaltraktes aufzuweiten, läuft das Lumen für den Führungsdraht innerhalb oder parallel des Ballonlumenens von einem eigenen Anschlussstück bis zur distalen Spitze.

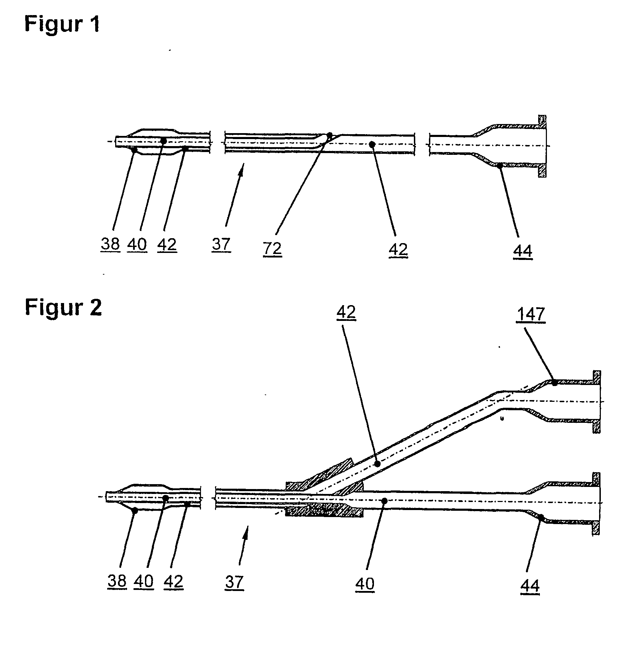

Die Figuren 1 und 2 zeigen Skizzen beider Kathetertypen.

Es ist erkennbar, dass Ballonkatheter nach einem Gebrauch mit bekannten Reinigungsvorrichtungen unter Einhaltung der geforderten hygienischen Bedingungen nicht aufzubereiten sind, um sie einer Wiederverwendung zuzuführen. Da Ballonkatheter je nach Ausführungsform in einem Preisbereich von etwa 400,-- bis 1.000,-- DM liegen, wäre eine Aufbereitung solcher Ballonkatheter jedoch wünschenswert.

Aus der WO 97 32610 A geht ein Apparat zur Desinfektion von medizinischen Geräten hervor, der eine Ultraschallwanne umfasst, in der sich eine Aufnahmewanne befindet. In der Aufnahmewanne ist ein Siebeinsatz zur Aufnahme von Medizinprodukten angeordnet. Ein solcher Apparat ist zur Reinigung von Medizinprodukten gedacht, die vollständig in eine Reinigungslösung eintauchbar sind. Ballonkatheter mit verschiedenen Lumina können mit einem derartigen Apparat nicht so aufbereitet werden, dass sie nach einer ersten Verwendung noch einmal verwendet werden können. Die Aufgabe der vorliegenden Erfindung besteht daher darin, eine Einrichtung zur hygienisch unbedenklichen Aufbereitung von Ballonkathetern zu schaffen, mit deren Hilfe Ballonkatheter so zu reinigen, desinfizieren und zu spülen sind, dass sie nach einer ersten Verwendung wenigstens noch einmal sterilisierbar und wiederverwendbar sind.
Diese Aufgabe wird durch eine Einrichtung zur Aufbereitung von Ballonkathetern mit den Merkmalen des Patenanspruches 1 gelöst. Die vor einer erneuten Verwendung der Katheter ebenfalls notwendigen Verfahrenschritte der Prüfung, Trocknung, ggf. Ballonfaltung, Verpackung und Sterilisation werden nicht durch die beschriebene Einrichtung erfüllt.

Der wesentliche Vorteil der vorliegenden Erfindung besteht darin, dass einmal, insbesondere zur Dilatation verwendete Ballonkatheter unter Einhaltung der geforderten hygienischen Bedingungen so aufbereitet werden können, dass sie nach den genannten weiteren Verfahrensschritten wenigstens einer Wiederverwendung zugeführt werden können. Dabei wird vorteilhafterweise eine geeignete Reinigung, Desinfektion und Spülung der kompliziert aufgebauten Ballonkatheter absolut sicher gestellt, indem über geeignete Vorrichtungen ein definierter und reproduzierbarer Kontakt zu den zu reinigendenden Flächen mit entsprechend geeigneten Medien sichergestellt und alle für den hygienischen Erfolg des Verfahrens relevanten physikalischen Parameter überwacht werden. Die Entkeimung und Spülung umfasst insbesondere die gesamte Außenseite des Katheters, die Innenfläche des Ballons und seines medienzuführenden Ballonlumens sowie die Innenfläche des Führungsdrahtlumens.

Vorteilhafterweise ist die erfindungsgemäße Einrichtung zur Aufbereitung von Ballonkathetern so ausgestaltet, dass eine gleichzeitige Aufbereitung von mehreren Ballonkathetern möglich ist. Die erfindungsgemäße Einrichtung kann daher besonders wirtschaftlich arbeiten. Dabei ist vorteilhafterweise sichergestellt, dass die parallel nebeneinander angeordneten Ballonkatheter berührungsfrei voneinander gehalten werden, sodass Kreuzkontaminationen durch direkten Kontakt vermieden werden können.

Weitere vorteilhafte Ausgestaltungen gehen aus den Unteransprüchen hervor.

Im folgenden werden die Erfindungen deren Ausgestaltungen im Zusammenhang mit den Figuren näher erläutert. Es zeigen:
- Figur 1: eine Skizze des Kathetertyps "Rapid-Exchange", synonym auch als "Fast-Exchange"-, "Monorail"- Katheter, Katheter nach "Bonzel", beziehungsweise Katheter der "Kurzdrahttechnik" bezeichnet;
- Figur 2: eine Skizze des Kathetertyps "Over-the-wire" synonym auch als "Katheter nach "Simpson", beziehungsweise Katheter der "Langdrahttechnik" bezeichnet;
- Figur 3: in schematischer Darstellung der Längsschnitt einer erfindungsgemäßen Einrichtung zur Aufbereitung von Ballonkathetern;
- Figur 4: eine Ansicht von oben auf das Siebeinsatz der Einrichtung der Figur 3;
- Figuren 5 bis 7: verschiedene Aufnahmeeinrichtungen zur Fixierung des Katheters auf dem Siebeinsatz;
- Figur 8: der Längsschnitt einer Vorrichtung zur Ankopplung an das Führungsdrahtlumen von Kathetern des Typs, "Rapid-Exchange" der Figur 1;
- Figur 9: eine Ansicht von oben auf die untere Dichtung der Vorrichtung der Figur 8 und auf einen darauf befindlichen Katheter;
- Figur 10: eine bevorzugte Ankopplung des Ballonanschlussteiles des Katheters; und
- Figur 11: in schematischer Darstellung eine Dosierungseinrichtung zur geregelten, redundant überwachten Dosierung einer Reinigungslösung und eines Desinfektionsmittels.

Im folgenden wird zunächst im Zusammenhang mit der Figur 81 der Aufbau eines an sich bekannten Ballonkatheters nach dem "Rapid-Exchange"-Typ näher erläutert.

Im wesentlichen besteht der Ballonkatheter 37 aus einem proximalen Abschnitt, an dessem einenEnde ein Anschlusssteil 44 angeordnet ist, an das eine Inflationsspritze zum Einbringen eines Inflationsmediums, beispielsweise eines Gemisches aus einem Kontrastmittel und Wasser, ansetzbar ist. Der proximale Abschnitt geht in einen distalen Abschnitt über, wobei der proximale Abschnitt und der distale Abschnitt ein Ballonlumen 42 umschließen, das vom Ballonanschlussteil 44 zum Ballon 38 reicht, der sich am freien Endbereich des distalen Abschnittes befindet. In den distalen Abschnitt mündet an einer Führungsdrahtaustrittstelle 72 ein Röhrchen ein, das ein Führungsdrahtlumen 40 umschließt. Dieses Führungsdrahtlumen 40 dient zum Hindurchschieben eines Führungsdrahtes, auf dem der Ballon 38 am Ende des distalen Abschnittes zum Ort einer Stenose verschiebbar ist.

Ballonkatheter des "Over-the-wire"-Typs gemäß Figur 2 besitzen zwei Anschlussstücke 44, 147 und zwei durchgängige Lumen für die Balloninflation 42 beziehungsweise für den Führungsdraht 40.

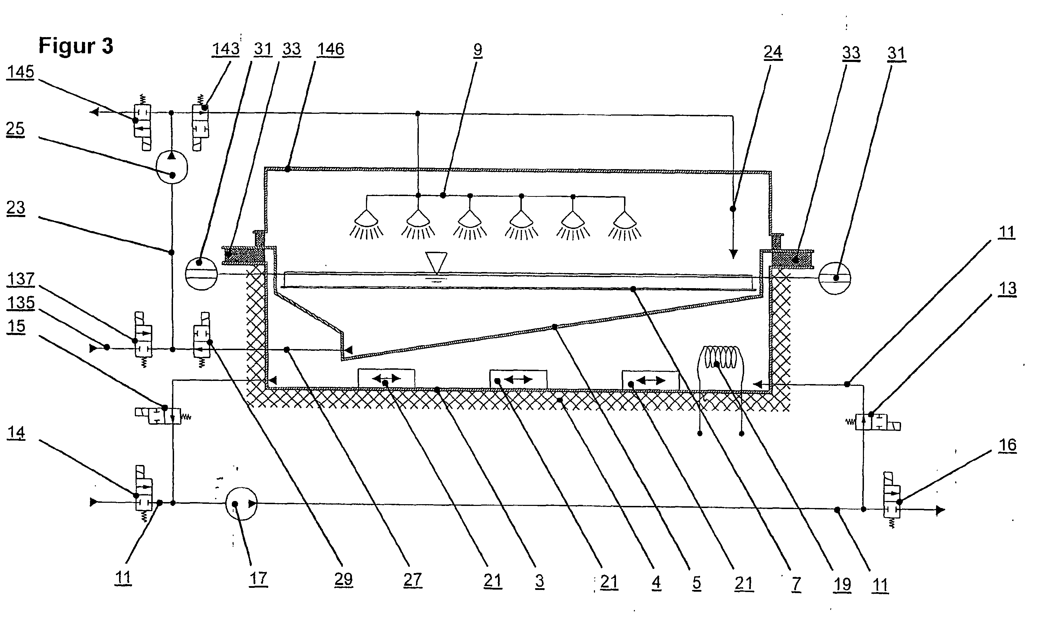

Gemäß Figur 3 besteht die vorliegende Einrichtung 1 zur Aufbereitung von Ballonkathetern im Wesentlichen aus einer äußeren Ultraschallwanne 3, einer inneren Aufnahmewanne 5, die in der Ultraschallwanne 3 angeordnet ist, einem in der Aufnahmewanne 5 gehaltenen Siebeinsatz 7 zur Aufnahme von Medizinprodukten, insbesondere von Ballonkathetern, und einer Sprühdüsenanordnung 9 innerhalb einer Abdeckung 146.

Der Ultraschallwanne 3 kann über eine Zufuhrleitung 11 und Ventil 14 ein Medium, beispielsweise Wasser zugeführt werden. Vorzugsweise wird das Wasser von der Zufuhrleitung 11 über ein weiteres Zufuhrventil 13, bei dem es sich vorzugsweise um ein elektromagnetisches Ventil handelt, der Ultraschallwanne 3 zugeführt. Das Wasser kann_über_ein. Abfuhrventil 15, bei dem es sich vorzugsweise ebenfalls um ein elektromagnetisches Ventil handelt, der Ultraschallwanne 3 entnommen und wieder der Zufuhrleitung 11 zugeführt werden. In der Zufuhrleitung 11 befindet sich zwischen dem Zufuhrventil 13 und dem Abfuhrventil 15 eine Umwälzpumpe 17, mit deren Hilfe das über das Abfuhrventil 15 entnommene Wasser umwälzbar und wieder über das Zufuhrventil 13 zuführbar ist. Auf diese Weise wird in der Ultraschallwanne 3, die über eine Heizeinrichtung 19 beheizbar ist, eine homogene Wärmeverteilung erreicht und wird bei Bedarf ein Austausch des Mediums ermöglicht. Die Zufuhr des Mediums beziehungsweise Wassers erfolgt bei geöffnetem Zufuhrventil 13, gesperrtem Abfuhrventil 15 und gesperrtem Ventil 16, das vorzugsweise mit der Zufuhrleitung 11 an einem Ort hinter der Umwälzpumpe 17 verbunden ist, über das Ventil 14. Bei der Heizeinrichtung 19 handelt es sich vorzugsweise um eine elektrische Heizeinrichtung. Andere Ventilpositionen und -arten, wie beispielsweise die Verwendung von 3/2-WegeVentilen anstatt der oben beschriebenen 2/2-Wege-Ventile, sind möglich, wenn durch ihre Anordnung die beschriebenen Funktionen der Medienzu- und -abführung sowie Umwälzung erfüllt sind.

In der Ultraschallwanne 3 sind Ultraschallsonden 21 beliebig positionierbar. Im vorliegenden Beispiel sind drei Ultraschallsonden 21 vorgesehen. Die separate Anordnung der getaucht anwendbaren Ultraschallsonden 21 in einer eigenen Wanne verringert vorteilhafterweise die von den zu reinigenden Kathetern und den dazu verwendeten Medien kontaminierten Oberflächen der Anlage und damit die Gefahr einer Keimverschleppung.

In der Ultraschallwanne 3 befindet sich die Aufnahmewanne 5, in die über eine Zufuhrleitung 23, in der sich eine Pumpe 25 befindet, ein Gemisch aus einer Reinigungslösung und einem Desinfektionsmittel über die Sprühdüsenanordnung 9 und/oder einen direkten Zulauf 24 zuführbar ist. Das Gemisch kann über einen Ablauf 27 der Aufnahmewanne 5 entnommen und über ein Ventil 29 wieder der Zufuhrleitung 23 zugeführt werden. Auf diese Weise kann das Gemisch im Kreis geführt werden. Vorzugsweise enthält dieser Umwälzkreislauf nicht dargestellte Filter, die dem Rückhalt vom Katheter entfernter Partikel dienen. Durch die schematisch dargestellte Pegelmesseinrichtungen 31 werden der Gemischpegel in der Aufnahmewanne 5 und der Wasserpegel in der Ultraschallwanne 3 vorzugsweise auf einem gleichen Niveau gehalten, wobei dafür Sorge getragen ist, dass dieses Niveau so hoch liegt, dass die auf dem Siebeinsatz 7 befindlichen Katheter 37 vollständig in das Gemisch eintauchen. Andere Ventilpositionen, wie beispielsweise die Verwendung von 3/2-Wege-Ventilen anstatt der in Figur 3 skizzierten 2/2-Wege-Ventile, sind möglich, wenn durch ihre Anordnung die beschriebenen Funktionen der Medienzu- und - abführung sowie Umwälzung erfüllt sind.

Vorzugsweise ist die in die Ultraschallwanne 3 hineinhängende Aufnahmewanne 5 an der oberen Seite der Ultraschallwanne 3 mit der Hilfe einer Dichtung 33 und Befestigungselementen (nicht dargestellt) dicht befestigt, damit verhindert wird, dass dann, wenn das Gewicht der Aufnahmewanne 5 mit den darin enthaltenen Medizinprodukten beziehungsweise Kathetern 37 kleiner ist als das Gewicht des aus der Ultraschallwanne 3 verdrängten Wassers, der entstehende Auftrieb die Position der Aufnahmewanne 5 nicht verändern kann.

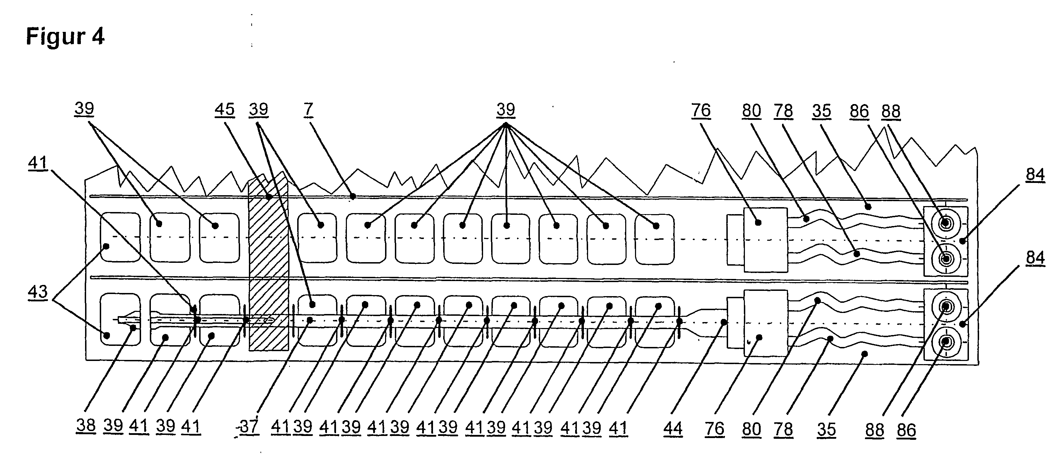

In der Aufnahmewanne 5 liegt der Siebeinsatz 7, auf dem in der nachfolgend im Zusammenhang mit der Figur 4 beschriebenen Weise die aufzubreitenden Katheter gehalten werden.

Die Figur 4 zeigt eine Aufsicht auf einen Ausschnitt des Siebeinsatzes 7, in dem vorzugsweise nebeneinander mehrere Aufnahmekanäle 35 für Katheter 37 angeordnet sind. Der Boden des Siebeinsatzes 7 umfasst in jedem Aufnahmekanal 35 hintereinander mehrere Durchbrüche 39, wobei jeweils zwischen zwei Durchbrüchen 39 Katheterfixierungen 41 angeordnet sein können. In den Katheterfixierungen 41 wird ein über die Durchbrüche 39 ausgelegter Katheter 37 beispielsweise über schwenk- und/oder steckbare Bügel oder Drähte oder über Gummiringe oder dergleichen festgehalten.

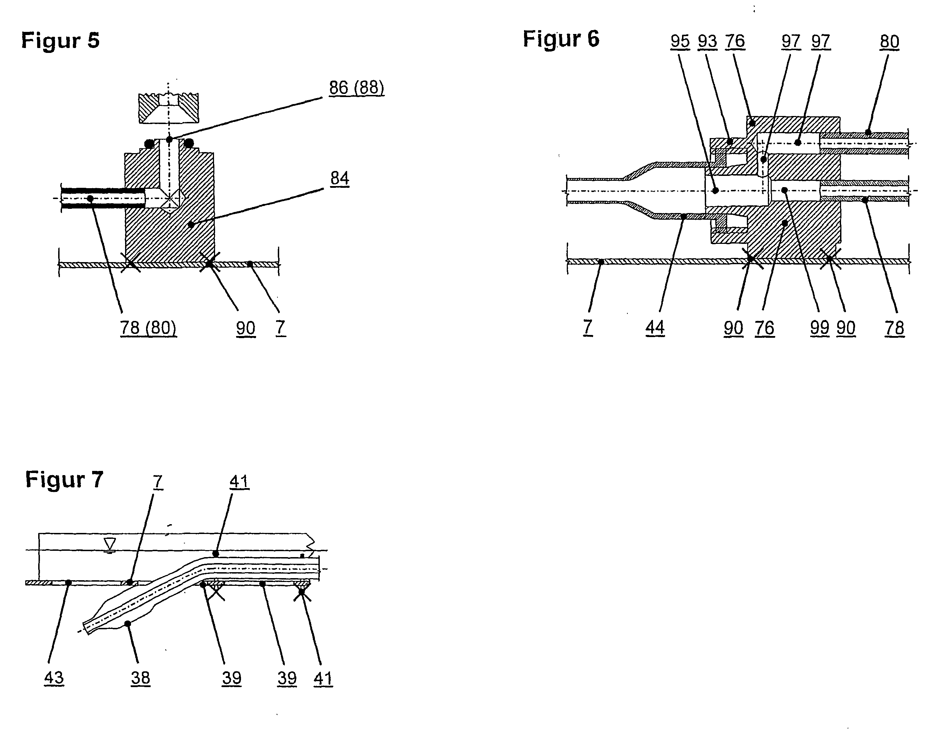

An einer Seite des Aufnahmekanals 35 befindet sich ein erster Durchbruch 43, der so bemessen ist, dass der Ballon 38 des Katheters 37 ausgehend von der Katheterfixierung 41 zwischen dem ersten Durchbruch 43 und dem nachfolgendem Durchbruch 39 oder zwischen weiteren nachfolgenden Durchbrüchen 39 schräg nach unten verläuft, sodass er schräg nach unten geneigt ist, damit seine Entlüftung durch Aufwärtssteigen und Absaugen von Luftblasen möglich ist. Dies ist in der Figur 7 näher dargestellt.

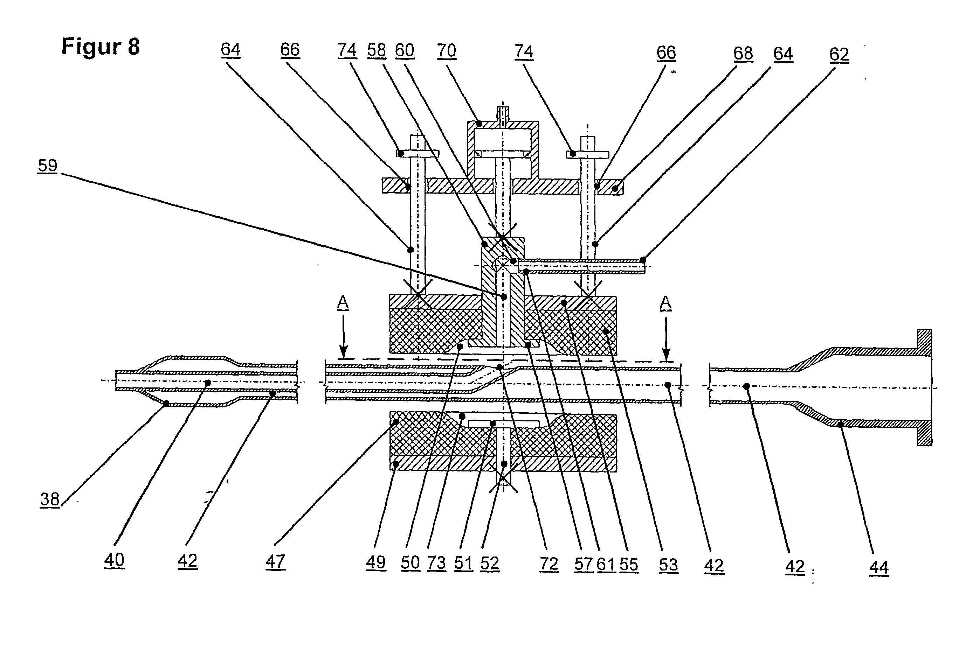

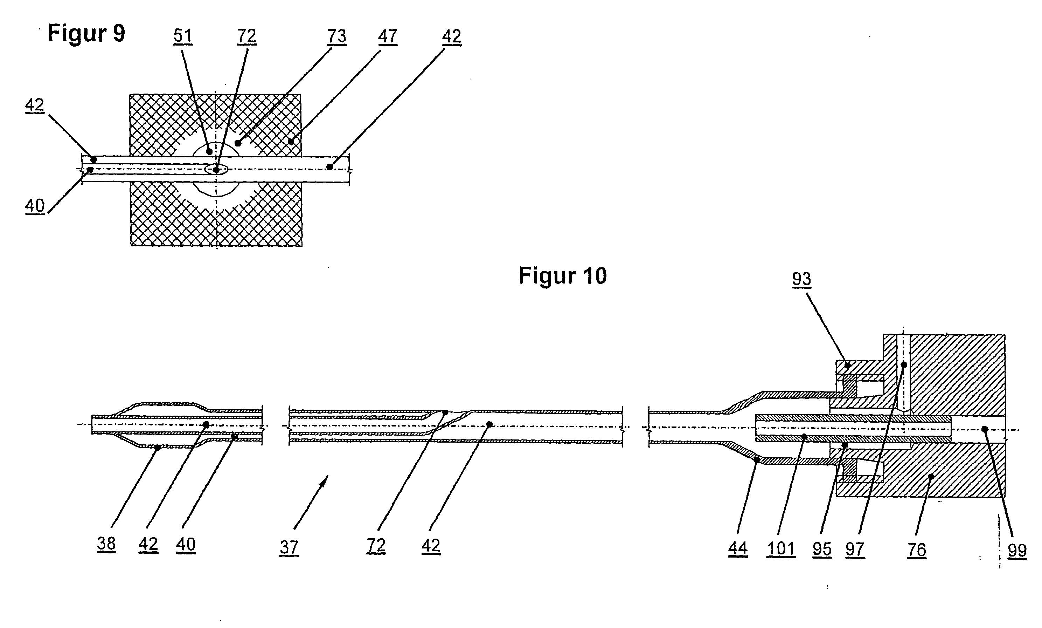

Vom ersten Durchbruch 43 zur anderen Seite des Aufnahmekanals 35 beabstandet befindet sich ein Bereich 45 zur Ankopplung des Führungsdrahtlumens 40 für den Führungsdraht an der Führungsdrahtaustrittsstelle 72 (siehe Figur 1) eines "Rapid-Exchange"-Katheters 37. Die Ausgestaltung dieses Bereiches wird nachfolgend im Zusammenhang mit den Figuren 8 und 9 näher erläutert. Bei Kathetern des "Over-the-wire"-Typs ist die nachfolgend beschriebene Ankopplungsvorrichtung, falls vorhanden, geöffnet, da eine direkte Verbindung der Zufuhrleitungen 62 (siehe Figur 8) mit dem genormten Anschlussstück der Führungsdrahtlumen 147 möglich ist.

Im Wesentlichen umfasst der Bereich 45 pro Kanal 35 eine unterhalb des Katheters 37 angeordnete erste elastische Dichtung 47, die beispielsweise aus EPDM-Moosgummi besteht. Diese erste elastische Dichtung 47 wird auf einer quer zu den Kanälen 35 verlaufenden Unterlage 49 festgehalten, wobei der mittlere Bereich 73 der Dichtung 47, die beispielsweise eine quadratische Form besitzt, durch einen Niederhalter 51 in Richtung auf die Unterlage 49 gezogen wird, sodass der mittlere Bereich 73 der Dichtung 47 eine weniger größe Dicke aufweist als der äußere Bereich der Dichtung 47. Darüber, das heißt also oberhalb des Katheters 37 angeordnet, befindet sich eine zweite Dichtung 53, die vorzugsweise entsprechend der ersten Dichtung 47 ausgebildet ist und an einer quer zu den Kanälen 35 verlaufenden, im Beispiel beweglichen Unterlage beziehungsweise Platte 55 befestigt ist. Der mittlere Bereich 50 der Dichtung 53 wird ebenfalls durch einen Niederhalter 57 in Richtung auf die Platte 55 gezogen, sodass seine Dicke kleiner ist als diejenige des äußeren Bereiches der Dichtung 53. Vorzugsweise we.ist der untere Niederhalter 51 die Form einer an einem Schaft 52 befestigten Niederhalterplatte auf, wobei der Schaft 52 mit der Hilfe eines Gewindes und einer Mutter oder dgl. einstellbar ist, sodass die Niederhalterplatte in einer vorgegeben Weise zur Bestimmung der Dicke des mittleren Bereiches der Dichtung 47 in Richtung auf die Unterlage 49 ziehbar ist. Entsprechend weist der obere Niederhalter 57 ebenfalls eine Niederhalterplatte auf, die an einem Schaft 58 befestigt ist, der in der Platte 55 einstellbar fixierbar ist. Durch den Schaft 58 verläuft eine senkrechte Bohrung 59, die sich an einer Seite durch die Niederhalteplatte nach außen öffnet und die an der anderen Seite über eine radial verlaufende Bohrung 60 zu einen seitlich an den Schaft 58 angeformten Anschlusselement 61 für eine Schlauchleitung 62 oder dgl. führt. Die Platte 55 weist vorzugsweise sich senkrecht zu ihrer Ebene erstreckende Führungsstäbe 64 auf, die durch Führungsbohrungen 66 verlaufen, die sich in einer oberhalb der Platte 55 befindlichen Führungsplatte 68 befinden. Auf diese Weise ist die Platte 55 und damit auch die Dichtung 53 derart bewegbar, dass sie in einer ersten Position gegen den Katheter 37 und die untere Dichtung 47 gedrückt wird, sodass die mittleren Bereiche 50 und 73 der Dichtungen 53 und 47 einen geschlossenen Raum bilden, indem sich die Öffnung 72 des Führungsdrahtlumens 40 des Katheters 37 befindet, und dass sie in einer zweiten Position, in der sich die obere Dichtung 53 oberhalb des Katheters 37 und der unteren Dichtung 47 befindet, sodass ein Katheter 37 zwischen die Dichtungen 47, 53 einlegbar oder aus diesen entnehmbar ist. Die Bewegung der oberen Dichtung 53 beziehungsweise der Platte 55 erfolgt vorzugsweise durch einen hydraulischen oder pneumatischen Mechanismus 70, der in der Figur 8 schematisch dargestellt ist. Dieser Mechanismus kann jedoch auch elektromagnetisch oder mechanisch (beispielsweise Zahnstange) ausgebildet sein.

Die Figur 9 zeigt eine Aufsicht entlang der Linie A - A der Figur 8 auf einen Katheter 37 und die untere Dichtung 47.

Um die untere Position der Dichtung 53 exakt anfahren zu können, befinden sich zur Wegbegrenzung beispielsweise an den Führungsstangen 64 an der der Platte 55 abgewandten Seite der Führungsplatte 68 Anschlagteile 74, die bei Erreichen der ersten Position an der Führungsplatte 68 anschlagen. Alternativ können Anschläge auf zwischen den Platten 55 und 49 oder innerhalb des Mechanismus 70 positioniert sein, sofern sie die Funktion einer Wegbegrenzung erfüllen.

Gemäß Figur 4 befinden sich am anderen Ende des Katheters 37 in der Längsrichtung des Kanals 35 bewegliche Aufnahmeteile 76, an die das Ballonanschlussteil 44 des Katheters 37 in der später näher erläuterten Weise, ankoppelbar ist. Vorzugsweise besitzen die Aufnahmeteile 76 die Form von in der Längsrichtung des Kanals 35 am Boden des Siebeinsatzes 7 verschiebbaren Blöcken, die gemäß Figur 4 über zwei Anschlussleitungen beziehungsweise Schläuche, nämlich eine elastische Zufuhrleitung 78 und eine elastische Abfuhrleitung 80 mit einem an dem Siebeinsatz 7 befestigten Blockteil 84 verbunden sind. Das Blockteil 84 weist ein Anschlusssteil 86 für die Zufuhrleitung 78 und ein Anschlussteil 88 für die Abfuhrleitung 80 auf. Die Zu- und -abfuhrleitungen 78, 80 stehen im Inneren des Aufnahmeteiles 76 gemäß Figur 6 jeweils über einen Durchgänge 99 bzw. 97 mit dem Anschluss zum Ballonanschlussteil 44 des Katheters 37 in Verbindung. Die Verbindung des Blockteiles 84 mit der Anlage ist zum Zweck der Entnahme/Einlage bestückter Siebeinsätze 7 trennbar ausgeführt. Die dazu notwendige, bewegliche Ankopplungsvorrichtung des Blockteils 84 ist beispielsweise über eine O-Ring-gedichtete Kegelverbindung 86 beziehungsweise 88, wie in Figur 5 dargestellt, sichergestellt. Die Bewegung dieser Dichtung erfolgt vorzugsweise mit einer Vorrichtung, die analog der bereits beschriebenen Bewegungsvorrichtung für die Dichtung 47 aufgebaut ist.

Gemäß Figur 6 und Figur 4 können die Aufnahmeteile 76 zur Anpassung an Katheter 37 unterschiedlicher Längen an verschiedenen Orten des Siebeinsatzes 7 beziehungsweise des Kanals 35 vorzugsweise dadurch fixiert werden, dass sie einfach in verschiedenen Bohrungen des Siebeinsatzes 7 mit der Hilfe von nicht dargestellten stabförmigen Steckteilen an der Position 90, z.B. mit Passstiften in Kombination mit Sicherungen, beispielsweise O-Ringen, fixiert werden. Die Variabilität der Position des Aufnahmeteils 76 ermöglicht vorteilhafterweise die Positionierung der Führungsdrahtaustrittsstelle 72 von "Rapid-Exchage"-Kathetern im Ankopplungsbereich 4 5 (Figur 4), auch wenn die Katheter verschiedene Längen besitzen.

Die Figur 6 zeigt einen vertikalen Schnitt durch das Aufnahmeteil 76. Die Verzweigung von zuführendem und abführendem Kanal sowie dem Kanal zum Anschluss des Ballons liegt vorzugsweise unmittelbar vor dem Ballonanschluss, damit der dem Ballon vorgelagerte Totraum auf ein Minimum reduziert wird (siehe Figur 6).

Im folgenden wird im Zusammenhang mit der Figur 10 eine bevorzugte Ausführungsform zur Verbindung des Anschlussteiles 44 des Katheters 37 mit dem an verschiedenen Orten im Kanal 35 befestigbaren Aufnahmeteil 76 erläutert. Das Aufnahmeteil 76 weist an seiner dem Katheter 37 zugewandten Seite ein Anschlusselement 93 auf (siehe Figur 10), an dem das Ballonanschlussteil 44 des Katheters 37 mechanisch fixierbar ist. Das Anschlusselement 93 besitzt einen Durchgang 95, der mit einem weiteren Durchgang 97 im Aufnahmeteil 76 in Verbindung steht, der wiederum in der bereits erläuterten Weise mit der Abfuhrleitung 80 (siehe Figur 6) verbunden ist. Die Zufuhrleitung 78 steht mit einem Durchgang 99 des Aufnahmeteiles 76 in Verbindung, der wiederum mit einem Kanüleneinsatz 101 verbunden ist, der in Richtung auf den Katheter 37 über das Ende des Anschlusselementes 93 in das Ballonanschlussteil 44 des Katheters 37 vorragt, wenn dieser mit dem Anschlusselement 93 verbunden ist. Dabei ragt das freie Ende des Kanüleneinsatzes 101 bis kurz vor den Übergang zwischen dem Ballonanschlussteil 44 und dem Ballonlumen 42 des Katheters 37. Durch diese Anordnung des Kanüleneinsatzes 101 wird erreicht, dass der Totraum im Ballonanschlussteil 44 bei der Zufuhr des Reinigungs- und Desinfektionsgemisches über den Durchgang 99 und den Kanüleneinsatz 101 soweit wie möglich überbrückt wird und nicht die Reinigung von kleinen Ballons behindernd in Erscheinung tritt.

Es wird darauf hingewiesen, dass die Befestigung des Ballonanschlussteiles 44 des Katheters 37 im Anschlusselement 93 und die Gestaltung des Kanüleneinsatzes 101 beliebig ausgestaltet sein kann. Vorzugsweise ist die Vorrichtung an genormte sogenannte "Luer-Lock"-Anschlüsse von Kathetern angepasst.

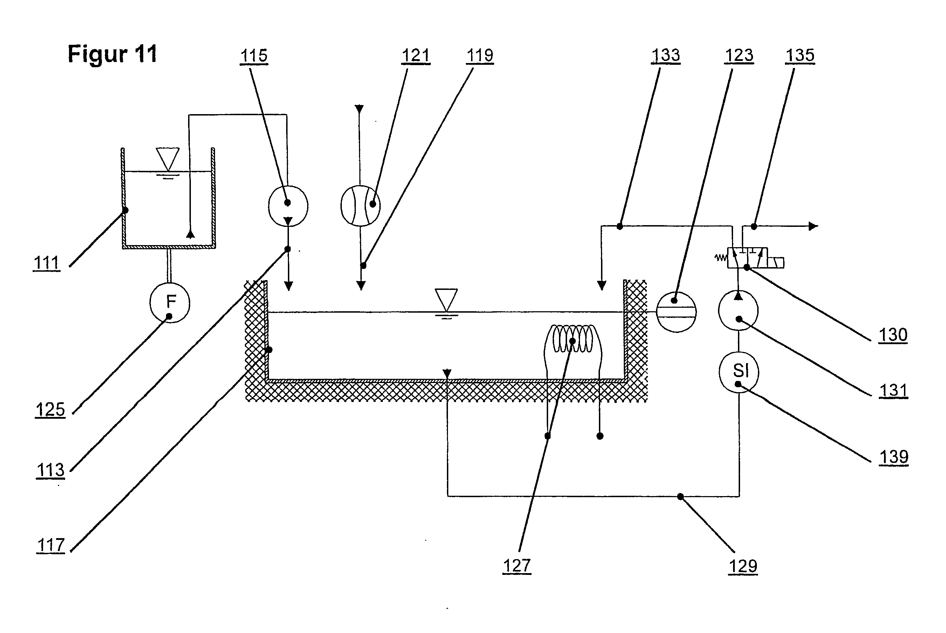

Nachfolgend wird im Zusammenhang mit der Figur 11 eine Anordnung zur geregelten, redundant überwachten Dosierung eines Gemisches aus Reinigungslösung und Desinfektionsmittel erläutert. Dabei wird das genannte Gemisch in der Form eines Konzentrates aus einem Behälter 111 über eine Leitung 113, in der sich eine Pumpe 115 befindet, abgepumpt und einem Gemischbehälter 117 zugeführt. Diesem Behälter 117 kann über eine Zulaufleitung 119 Wasser zugeführt werden. In der Zulaufleitung 119 befindet sich vorzugsweise ein Volumenstromsensor 121. Der Pegel im Gemischbehälter 117 wird durch einen Füllstandsensor 123 angezeigt.

Die Pumpe 115 ist vorzugsweise eine Pumpe, die mit einem konstanten Volumenstrom fördert beziehungsweise abpumpt. Der Behälter 111 befindet sich vorzugsweise auf einer Wägeeinrichtung 125, mit deren Hilfe die Menge des entnommenen Konzentrats regelbar ist.

Das im Gemischbehälter 117 befindliche Gemisch ist durch eine schematisch dargestellte Heizeinrichtung 127, die vorzugsweise elektrisch arbeitet, auf eine vorgegebene Temperatur erwärmbar. Das Konzentrat/Wasser-Gemisch wird dem Behälter 117 über eine Ablaufleitung 129 mit der Hilfe einer Pumpe 131 entnommen und über eine Zulaufleitung 135 der vorliegenden Einrichtung beziehungsweise der im Zusammenhang mit der Figur 3 bereits erläuterten Zufuhrleitung 23 beispielsweise über ein Zufuhrventil 137 (Figur 3) zugeführt. In der Ablaufleitung 129 befindet sich vorzugsweise ein Leitwertsensor 139 zur Bestimmung des elektrischen Leitwertes des dem Behälter 117 entnommenen Gemisches.

Vorzugsweise befindet sich am Ende der Ablaufleitung 129 ein Umschaltventil 130, beispielsweise ein 3/2-Wege-Ventil, durch das die Umschaltung zwischen der Rücklaufleitung 133 und der Zulaufleitung 135 erfolgen kann.

Die Konzentratentnahme aus dem Behälter 111 erfolgt durch Ansteuern der Pumpe 115, die mit einem konstanten Volumenstrom fördert, solange, bis die gewünschte Zielmenge, gemessen durch die Wägeeinrichtung 125, entnommen ist. Die redundante Überwachung erfolgt über die Laufzeit der mit dem konstanten Volumenstrom fördernden Pumpe und vorzugsweise zusätzlich über den durch den Leitwertsensor 139 in der Ablaufleitung 129 gemessenen Leitwert, der als Maß für das gewünschte Konzentrat/Wasser-Gemisch, sofern dieses ein Elektrolyt ist, nutzbar ist.

Im folgenden wird die Arbeitsweise zum Aufbereiten von Kathetern 37 mit der vorliegenden Einrichtung näher erläutert. Das dem Behälter 117 über die Zulaufleitung 135 (Figur 11) entnommene und über das Zufuhrventil 137 der Zufuhrleitung 23 (Figur 3) bei geschlossenem Ventil 29 im Ablauf 27 zugeführte Konzentrat/Wasser-Gemisch wird durch die von der Zulaufpumpe 25 bei geöffnetem Ventil 143 und geschlossenem Ventil 145 der Zufuhrleitung 23 zugeführt und zur Reinigung der Außenflächen der auf dem Siebeinsatz 7 befindlichen Katheter der Sprühdüsenanordnung 9 zugeführt.

Gleichzeitig wird über den Zulauf 24 das, Konzentrat/Wasser-Gemisch direkt zu den über die Blockteile 84 der Kanäle 35 und die entsprechenden Zufuhrleitungen 78 den einzelnen Aufnahmeteilen 76 der Kanäle 35 zugeführt (Figur 4). Gemäß Figur 10 wird in den Aufnahmeteilen 76 das Gemisch über die Durchgänge 99 und die Kanüleneinsätze 101 unter Überbrückung des Totraumes direkt oder über Zwischenspeicher in das Ballonlumen 42 der Katheter 37 zur Spülung der Innenfläche des Ballonlumens 42 und der Innenfläche des Ballons 38 der Katheter 37 zugeführt. Die Entleerung des Ballons erfolgt durch Anlegen eines Unterdruckes am Anschluss 44 oder durch Anlegen eines äußeren Druckes am Ballon. Den Zufuhrleitungen 78 und den Abfuhrleitungen 80 sind zu diesem Zweck nicht dargestellte Druck- beziehungsweise Unterdruckspeicher und Ventile vorgelagert. Die Reinigungswirkung ist wegen der Überbrückung des Totraumes im Ballonanschlussteil 44 durch den Kanüleneinsatz 101 besonders wirkungsvoll. Die Reinigung, Desinfektion und Spülung der Ballons 38 und Ihrer Lumen 42 erfolgt durch zyklische Füllung und Entleerung. Die Durchströmung des Führungsdrahtlumens 40 kann kontinuierlich erfolgen. Sowohl die Ballondrücke, als auch die Volumenströme durch die Führungadrahtlumina werden vorzugsweise einzeln durch Sensoren überwacht.

Da die Pegel des Gemisches in der Aufnahmewanne 5 und des Wassers in der Ultraschallwanne 3 auf ein gemeinsames Niveau eingeregelt werden, das sich oberhalb der auf dem Siebeinsatz 7 angeordneten Katheter 37 befindet, erfolgt eine Unterstützung der Reinigung der Katheter 37 durch die durch die Ultraschallsonden 21 erzeugten Ultraschallwellen, die über das Wasser und das Gemisch auf die Katheter 37 übertragen werden und oberflächliche Kontaminationen abtragen. Gleichzeitig wird das Gemisch in der Aufnahmewanne 5 auf der Temperatur des Wassers in der beheizten Ultraschallwanne 3 gehalten und dient als Wärmespeicher hoher Kapazität.

In der bereits erläuterten Weise erfolgt gleichzeitig die Reinigung und Desinfektion der Innenflächen des Führungsdrahtlumens 40 der "Rapid-Exchange"-Katheter 37 dadurch, dass gemäß Figuren 7 und 8 bei geschlossenen Dichtungen 47 und 53 über die Schlauchleitung 62 und dem direkten Zulauf 24 (Figur 3) das Gemisch vorzugsweise gleichzeitig mit dem Einbringen des Gemisches in das Ballonlumen 42 der Katheter 37 eingebracht wird. Zu diesem Zweck stehen die Schlauchleitungen 62 vorzugsweise direkt mit dem Zulauf 24 in Verbindung. Katheter des sogenannten "Over-the-wire"-Typs werden am vorhandenen Führungsdrahtanschluss direkt mit der Schlauchleitung 62 verbunden.

Die Reinigung und Desinfektion (Keimabtötung) der Außenfläche des Katheters 37 und des Ballons 38 über die Sprühdüsenanordnung 9 und unterstützt durch Ultraschall, der Innenfläche des Ballonlumens 42 und des Ballons 38 und der Innenfläche des Führungsdrahtlumens 40 über die Dichtungen 47, 53 bei "Rapid-Exchange"-Kathetern beziehungsweise direkt über den Anschluss 147 bei "Over-the-wire"-Kathetern erfolgt im Kreislauf durch Pumpen des Gemisches durch die Pumpe 25 bei geöffneten Ventilen 143 und 141 und bei geschlossenen Ventilen 137 und 145.

Es wird darauf hingewiesen, dass es sich bei allen genannten Ventilen vorzugsweise um elektromagnetische Ventile handelt. Andere Ventilanordnungen, als die beispielsweise skizzierten, sind möglich, wenn diese die beschriebenen Funktionen erfüllen.

Die Aufnahmewanne 5 weist zweckmäßigerweise eine als Ablauf wirkende Bodenwand auf, die zum Auslauf 27 hin geneigt ist. Um einen Wärmeverlust aus der Ultraschallwanne 3 möglichst klein zu halten, kann diese außen mit einem Isoliermantel 4 versehen sein, wie dies in der Figur 3 schematisch dargestellt ist.

## Patentansprüche

1. Einrichtung zur Aufbereitung wenigstens eines Ballonkatheters (37) mit einem Ballonanschlussteil (44) an einem proximalen Ende, welches über ein Ballonlumen (42) mit einem am distalen Endbereich des Katheters (37) angeordneten Ballon (38) verbunden ist, und mit einem Führungsdrahtlumen (40), das zu einer Führungsdrahtaustrittstelle (72) oder zu einem eigenen Führungsdrahtanschluss (147) führt, wobei eine, ein Fluid enthaltende Ultraschallwanne (3) zur Ultraschallerzeugung in dem Fluid vorgesehen ist, in der sich eine Aufnahmewanne (5) befindet, die in das Fluid eintaucht, wobei in der Aufnahmewanne (5) ein Siebeinsatz (7) zur Aufnahme des wenigstens einen Ballonkatheters (37) gehalten ist, wobei dem Ballon (38) des Ballonkatheters (37) von einem Aufnahmeteil (76) über das Ballonanschlußteil (44) und das Ballonlumen (42) Gemisch aus einer Reinigungs- und Desinfektionslösung zuführbar und durch Anlegen eines Unterdrucks an das Ballonanschlußteil (44) oder durch Ausüben eines Druckes auf den Ballon (38) aus dem Ballon (3) und dem Ballonlumen (42) entnehmbar ist, und wobei Gemisch der Reinigungs-und Desinfektionslösung über die Führungsdrahtaustrittstelle (72) bzw. den Führungsdrahtanschluss (147) dem Führungsdrahtlumen (40) zuführbar ist, **dadurch gekennzeichnet, dass** oberhalb des Siebeinsatzes (7) eine Sprühdüsenanordnung (9) vorgesehen ist, der Gemisch der Reinigungs- und Desinfektionslösung zuführbar ist und dass die Ultraschallwanne (3) durch eine die Sprühdüsenanordnung (9) überdeckende Abdeckung (146) dicht abdeckbar ist.

2. Einrichtung nach Anspruch 1, **dadurch gekennzeichnet, dass** der Ultraschallwanne (3) über eine Zufuhrleitung (11) das Fluid, vorzugsweise Wasser, zuführbar ist, dass das Fluid aus der Ultraschallwanne (3) entnehmbar und über eine Umwälzpumpe (17) wieder der Zufuhrleitung (11) zuführbar ist.

3. Einrichtung nach Anspruch 2 **dadurch gekennzeichnet, dass** sich zwischen der Ultraschallwanne (3) und der Umwälzpumpe (17) ein elektromagnetisches Abfuhrventil (15) und zwischen der Umwälzpumpe (17) und der Ultraschallwanne (3) ein Zufuhrventil (13) befinden.

4. Einrichtung nach einem der Ansprüche 2 bis 3, **dadurch gekennzeichnet, dass** in der Ultraschallwanne (3) wenigstens eine Ultraschallsonde (21) in das Fluid eintauchend vorgesehen ist.

5. Einrichtung nach Anspruch 4, **dadurch gekennzeichnet, dass** mehrere Ultraschallsonden (21) angeordnet sind.

6. Einrichtung nach einem der Ansprüch 1 bis 5, **dadurch gekennzeichnet, dass** eine Heizeinrichtung (19) zur Erwärmung des in der Ultraschallwanne (3) enthaltenen Fluids auf eine vorgegebene Temperatur angeordnet ist.

7. Einrichtung nach einem der Ansprüche 1 bis 6,
**dadurch gekennzeichnet, dass** die in die Ultraschallwanne (3) hineinhängende Aufnahmewanne (5) oberseitig mit der Hilfe einer Dichtung (33) dicht am oberen Rand an der Oberseite der Ultraschallwanne (3) befestigt ist, so dass die Aufnahmewanne (5) in dem Fluid der Ultraschallwanne (3) keinen Auftrieb erfährt.

8. Einrichtung nach einem der Ansprüche 1 bis 7,
**dadurch gekennzeichnet, dass** auf der der Sprühdüsenanordnung (9) zugewandten Oberfläche des Siebeinsatzes (7) wenigstens ein Aufnahmekanal (35) für einen Katheter (37) angeordnet ist, dass der Aufnahmekanal (35) in seiner Längsrichtung hintereinander mehrere Durchbrüche (39) in dem Siebeinsatz (7) aufweist, dass der Aufnahmekanal (35) einen Bereich (45) zur Ankoppelung des Führungsdrahtlumens (40) und zum Zuführen von Gemisch der Reinigungs- und Desinfektionslösung aufweist, und dass an einer Seite des Aufnahmekanals (35) des Aufnahmeteil (76) angeordnet und mit dem Ballonanschlußteil (44) des Katheters (37) dicht verbindbar ist, derart, dass dem Ballonlumen (42) des Katheters (37) die Reinigungs- und Desinfektionslösung zuführbar und entnehmbar ist.

9. Einrichtung Anspruch 8, **dadurch gekennzeichnet, dass** an der anderen Seite des Aufnahmekanals (35) ein erster Durchbruch (43) vorgesehen und so bemessen ist, dass der Ballon (38) des Katheters (37), ausgehend von einer Fixiereinrichtung (41) zwischen den dem ersten Durchbruch (43) nachfolgenden beiden Durchbrüchen (39) unter dem dem ersten Durchbruch (43) benachbarten Durchbruch (39) und dem ersten Durchbruch (43)) schräg nach unten in Richtung auf die der Sprühdüsenanordnung (9) abgewandte Seite verläuft und somit schräg nach unten geneigt ist, damit seine Entlüftung durch Aufwärtssteigen und Absaugen von Luftblasen möglich ist.

10. Einrichtung nach Anspruch 8 oder 9, **dadurch gekennzeichnet, dass** in dem Siebeinsatz (7) nebeneinander mehrere parallel zu einander verlaufende Aufnahmekanäle (35) angeordnet sind.

11. Einrichtung nach einem der Ansprüche 8 bis 10, **dadurch gekennzeichnet, dass** der Bereich eine unterhalb eines Katheters (37) angeordnete erste elastische Dichtung (47), die auf einer quer zum Kanal verlaufenden Unterlage (49) befestigt ist, aufweist, dass in der ersten Dichtung (47) ein erster vertiefter Bereich (73) angeordnet ist, dass oberhalb der ersten Dichtung (47) und des Katheters (37) eine zweite Dichtung (53) angeordnet und an einer quer zum Aufnahmekanal (37) verlaufenden Platte (55) befestigt ist, dass in der zweiten Dichtung (53) ein zweiter vertiefter Bereich (50) angeordnet ist, dass die zweite Dichtung (53) und die erste Dichtung (47) so gegeneinander drückbar sind, dass die Führungsdrahtaustrittsstelle des Katheters (37) in einem von dem ersten und zweiten vertieften Bereich gebildeten abgedichteten Raum enthalten ist, in den zum Spülen des Führungsdrahtlumens (40) des Katheters (37) Gemisch des Reinigungs- und Desinfektionslösung einführbar ist.

12. Einrichtung nach Anspruch 11, **dadurch gekennzeichnet, dass** der erste vertiefte Bereich (73) in der ersten Dichtung (47) **dadurch** gebildet ist, dass der entsprechende Dichtungsbereich durch einen ersten Niederhalter (51) in Richtung auf die Unterlage (49) gezogen wird.

13. Einrichtung nach Anspruch 11 oder 12, **dadurch gekennzeichnet, dass** der zweite vertiefte Bereich (50) in der zweiten Dichtung (53) **dadurch** gebildet ist, dass der entsprechende Dichtungsbereich durch einen zweiten Niederhalter (57) in Richtung auf die Platte (55) gezogen wird.

14. Einrichtung nach Anspruch 13, **dadurch gekennzeichnet, dass** der zweite Niederhalter (57) einen durch die zweite Dichtung (53) verlaufenden Schaft (58) umfaßt, in dem eine Bohrung (59) angeordnet ist, über die Gemisch des Reinigungs- und Desinfektionslösung in den von dem ersten und zweiten vertieften Bereich gebildeten Raum einführbar ist.

15. Einrichtung nach einem der Ansprüche 8 bis 14, **dadurch gekennzeichnet, dass** die Unterlage (49) und die daran befestigte erste Dichtung (47) unterhalb eines Katheters (37) fest an den Siebeinsatz (7) angeordnet ist, dass die zweite Dichtung (53) durch einen Mechanismus (70) senkrecht zur Ebene des Aufnahmekanals (37) zum Öffnen der ersten und zweiten Dichtung und zum Einlegen eines Katheters (37) sowie zum Schließen der ersten und zweiten Dichtung und zum Bilden des Raumes bewegbar ist.

16. Einrichtung nach einem der Ansprüche 8 bis 17, **dadurch gekennzeichnet, dass** das Aufnahmeteil (76) die Form eines Blockteiles (84) aufweist, das in der Längsrichtung des Aufnahmekanals (35) an unterschiedlichen Orten des Siebeinsatzes (7) zur Anpassung an unterschiedliche Längen von Kathetern (37) befestigbar ist, dass das Ballonanschlußteil (44) eines Katheters (37) an einem Anschlußelement (93) des Aufnahmeteils (76) befestigbar ist, dass das Anschlußelement (93) einen Durchgang (95) aufweist, der mit einem weiteren Durchgang (97) im Aufnahmeteil (76) in Verbindung steht, der mit einer Abfuhrleitung (80) für die Reinigungs- und Desinfektionslösung verbindbar ist, und dass eine Zufuhrleitung (78) für Gemisch des Reinigungs- und Desinfektionslösung mit einem weiteren Durchgang (99) des Aufnahmeteiles in Verbindung steht, der mit dem Durchgang (95) verbunden ist.

17. Einrichtung nach Anspruch 16, **dadurch gekennzeichnet, dass** die Zufuhrleitung (78) mit einem Kanüleneinsatz (101) verbunden ist, der durch den Durchgang (95) in den Ballonanschluß (44) hineinverläuft, so dass sein freies Ende kurz vor dem Übergang zwischen dem Ballonanschlußteil (44) und dem Ballonlumen (42) des Katheters (37) angeordnet ist, so dass der Totraum im Ballonanschlussteil (44) bei der Zufuhr der Reinigungs- und Desinfektionslösung soweit wie möglich überbrückt ist.

18. Einrichtung nach einem der Ansprüche 8 bis 17, **dadurch gekennzeichnet, dass** das Aufnahmeteil (76) einen weiteren Anschluss zum Verbinden mit dem Führungsdrahtanschluss (147) und zum Zuführen von Gemisch der Reinigungs- und Desinfektionslösung aufweist.

19. Einrichtung nach einem der Ansprüche 1 bis 18, **dadurch gekennzeichnet, dass** ein Gemisch aus eine Reinigungs- und Desinfektionsmittel einem Behälter (111) entnehmbar und einem Gemischbehälter (117) zuführbar ist, dem über eine Zulaufleitung (119) Wasser zuführbar ist und dass das im Gemischbehälter (117) erzeugte Gemisch des Reinigungs- und Desinfektionslösung aus dem Gemischbehälter (117) der Spritzdüsenanordnung (9) sowie dem Aufnahmeteil (76) zuführbar ist.

20. Einrichtung nach Anspruch 19, **dadurch gekennzeichnet, dass** das im Gemischbehälter (117) enthaltene Gemisch des Reinigungs - und Desinfektionslösung durch eine Heizeinrichtung (127) des Gemischbehälters (117) auf eine vorbestimmte Temperatur erwärmbar ist.

21. Einrichtung nach einem der Ansprüche 1 bis 20, **dadurch gekennzeichnet, dass** zur Regelung des Pegels der Reinigungs- und Desinfektionslösung in der Aufnahmewanne (5) ein Pegelsensor vorgesehen ist, des den Pegel so regelt, dass der bzw. die auf dem Siebeinsatz (7) befindlichen Katheter (37) in die Reinigungs-und Desinfektionslösung voll eintauchen.

22. Einrichtung nach einem der Ansprüche 19 bis 21, **dadurch gekennzeichnet, dass** die Mischung des Reinigungs- und Desinfektionslösung **dadurch** erfolgt, dass die dem Behälter (111) über eine Pumpe (115) entnommene Menge des Gemisches durch eine das Gewicht des Behälters (111) ermittelnde Wägeeinrichtung (125) ermittelt und solange entnommen wird, bis im Gemischbehälter (117) die gewünschte Zielmischung aus Wasser und dem Gemisch entsteht.

23. Einrichtung nach Anspruch 22, **dadurch gekennzeichnet, dass** die Pumpe (115) mit einem konstanten Volumenstrom fördert und dass die Mengenentnahme aus dem Behälter (111) durch Erfassung der Laufzeit der Pumpe ermittelt bzw. überwacht wird.

24. Einrichtung nach Anspruch 22 oder 23, **dadurch gekennzeichnet, dass** die Mengenentnahme aus dem Behälter (111) durch einen in einer Ablaufleitung des Gemischbehälters (117) angeordneten Leitwertsensor (139) ermittelt bzw. überwacht wird.

25. Einrichtung nach einem der Ansprüche 19 bis 24, **dadurch gekennzeichnet, dass** die Reinigungs- und Desinfektionslösung aus dem Gemischbehälter (117) der Sprühdüsenanordnung (9) und dem bzw. den Aufnahmeteilen (76) zuführbar und aus der Aufnahmewanne ((5) in den Gemischbehälter (117) zurückführbar ist.

26. Einrichtung nach einem der Ansprüche 8 bis 25, **dadurch gekennzeichnet, dass** zwischen benachbarten Durchbrüchen (39) weitere Katheterfixierungen (41) vorgesehen sind, so dass ein über die Durchbrüche (39) in dem Aufnahmekanal (35) ausgelegter Katheter (37) in den weiteren Katheterfixierungen (41) fixierbar ist.

## Claims

1. A device for the preparation of at least one balloon catheter (7) having a balloon connection part (44) at a proximal end, which is connected via a balloon lumen (42) with a balloon (38) disposed at the distal end region of the catheter (37), and having a guide wire lumen (40), which leads to a guide wire outlet point (72) or to its own guide wire connection (147),
wherein an ultrasonic vat (3) containing a fluid is provided for ultrasonic generation in the fluid, in which a receiving vat (5) is situated, into which the fluid dips, wherein a screen insert (7) is held in the receiving vat (5) to receive the at least one balloon catheter (37),
wherein mixture from a cleaning and disinfecting solution can be supplied to the balloon (38) of the balloon catheter (37) from a housing part (76) via the balloon connection part (44) and the balloon lumen (42) and by applying a vacuum to the balloon connection part (44) or by exerting a pressure on the balloon (38) can be removed from the balloon (3) and the balloon lumen (42),
and wherein mixture of the cleaning and disinfecting solution can be supplied via the guide wire outlet point (72) or the guide wire connection (147) to the guide wire lumen (40),
**characterised in that** above the screen insert (7) is provided a spay nozzle configuration (9), to which mixture of the cleaning and disinfecting solution can be supplied and **in that** the ultrasonic vat (3) can be tightly covered by a cover (146) covering the spay nozzle configuration (9).

2. A device according to Claim 1,
**characterised in that** the fluid, preferably water, can be supplied to the ultrasonic vat (3) via a supply line (11),
**in that** the fluid can be removed from the ultrasonic vat (3) and can be supplied again via a circulating pump (17) to the supply line (11).

3. A device according to Claim 2,
**characterised in that** an electromagnetic delivery valve (15) is situated between the ultrasonic vat (3) and the circulating pump (17) and a supply valve (13) is situated between the circulating pump (17) and the ultrasonic vat (3) .

4. A device according to one of Claims 2 to 3,
**characterised in that** at least one ultrasonic probe (21) dipping into the fluid is provided in the ultrasonic vat (3) .

5. A device according to Claim 4,
**characterised in that** several ultrasonic probes (31) are provided.

6. A device according to one of Claims 1 to 5,
**characterised in that** a heating device (19) is provided for heating the fluid contained in the ultrasonic vat (3) to a predetermined temperature.

7. A device according to one of Claims 1 to 6,
**characterised in that** the receiving vat (5) hanging down into the ultrasonic vat (3) is tightly fixed at the upper side by means of a seal (33) to the upper edge at the upper side of the ultrasonic vat (3), so that the receiving vat (5) does not experience any lift in the fluid of the ultrasonic vat (3).

8. A device according to one of Claims 1 to 7,
**characterised in that** at least one housing duct (35) for a catheter (37) is disposed on the surface of the screen insert (7) facing the spray nozzle configuration (9),
**in that** the housing duct (35) in its longitudinal direction comprise several openings (39) one behind the other in the screen insert (7),
**in that** the housing duct (35) comprises a region (45) for coupling the guide wire lumen (40) and for supplying mixture of the cleaning and disinfecting solution,
and **in that** the housing part (76) is disposed on one side of the housing duct (35) and can be tightly connected to the balloon connection part (44) of the catheter (37) in such a manner that the cleaning and disinfecting solution can be supplied to and removed from the balloon lumen (42) of the catheter (37).

9. A device according to Claim 8,
**characterised in that** a first opening (43) is provided on the other side of the housing duct (35) and has such dimensions that the balloon (38) of the catheter (37), starting from a fixing device (41) between the two openings (39) following the first opening (43) beneath the opening (39) adjacent to the first opening (43) and the first opening (43), runs obliquely downwards in the direction of the side remote from the spray nozzle configuration and thus is inclined obliquely downwards, so that its deaeration is possible by the rising and extraction of air bubbles by suction.

10. A device according to Claim 8 or 9,
**characterised in that** several housing ducts (35) running mutually parallel are provided in the screen insert (7).

11. A device according to one of Claims 8 to 10,
**characterised in that** the region comprises a first elastic seal (47) disposed beneath a catheter (37), which is fixed on a base (49) running transversely to the duct,
**in that** in the first seal (47) a first recessed region (73) is provided,
**in that** above the first seal (47) and the catheter (37) a second seal (53) is provided and is fixed to a plate (55) running transversely to the housing duct (37),
**in that** a second recessed region (50) is provided in the in the second seal (53),
**in that** the second seal (53) and the first seal (47) can be pressed against each other so that the guide wire outlet point of the catheter (37) is contained in a sealed space formed by the first and the second recessed regions, in which space mixture of the cleaning and disinfecting solution can be introduced for rinsing the guide wire lumen (40) of the catheter (37).

12. A device according to Claim 11,
**characterised in that** the first recessed region (73) in the first seal (47) is formed by the corresponding sealing region being pulled by a first pressure pad (51) towards the base (49).

13. A device according to Claim 11 or 12,
**characterised in that** the second recessed region (50) in the second seal (53) is formed by the corresponding sealing region being pulled by a second pressure pad (57) towards the plate (55).

14. A device according to Claim 13,
**characterised in that** the second pressure pad (57) comprises a shaft (58) running through the second seal (53), in which a bore (59) is provided, via which mixture of the cleaning and disinfecting solution can be introduced into the space formed by the first and second recessed regions.

15. A device according to one of Claims 8 to 14,
**characterised in that** the base (49) and the first seal (47) fixed thereto is disposed beneath a catheter (37) securely to the screen insert (7),
**in that** the second seal (53) can be moved by a mechanism (70) perpendicularly to the plane of the housing duct (37) for opening the first and second seal and for the insertion of a catheter (37) and also for closing the first and second seals and for forming the space.

16. A device according to one of Claims 8 to 17,
**characterised in that** the housing part (76) takes the form of a block part (84), which can be fixed in the longitudinal direction of the housing duct (35) at different sites of the screen insert (7) for adaptation to different lengths of catheters (37),
**in that** the balloon connection part (44) of a catheter (37) can be fixed to a connection element (93) of the housing part (76),
**in that** the connection element (93) comprises a passage (95), which communicates with another passage (97) in the housing part (76), which can be connected to a discharge line (80) for the cleaning and disinfecting solution,
and **in that** a supply line (78) for mixture of the cleaning and disinfecting solution communicates with another passage (99) of the housing part, which is connected to the passage (95).

17. A device according to Claim 16,
**characterised in that** the supply line (78) is connected to a cannula insert (101) which runs through the passage (95) into the balloon connection (44), so that its free end is disposed a short distance in front of the transition between the balloon connection part (44) and the balloon lumen (42) of the catheter (37), so that the dead space in the balloon connection part (44) is bridged as far as possible during the supply of the cleaning and disinfecting solution.

18. A device according to one of Claims 8 to 17,
**characterised in that** the housing part (76) comprises a further connection for connection with the guide wire connection (147) and for the supply of mixture of the cleaning and disinfecting solution.

19. A device according to one of Claims 1 to 18,
**characterised in that** a mixture of a cleaning and disinfecting solution can be removed from a reservoir (111) and can be supplied to a mixture tank (117), to which water can be supplied via an inlet line (119)
and **in that** the mixture of the cleaning an disinfecting solution produced in the mixture reservoir (117) can be supplied from the mixture reservoir (117) to the spray nozzle configuration (9) and also the housing part (76).

20. A device according to Claim 19,
**characterised in that** the mixture of the cleaning and disinfecting solution contained in the mixture reservoir (117) can be heated by a heating device (127) of the mixture reservoir (117) to a predetermined temperature.

21. A device according to one of Claims 1 to 20,
**characterised in that** to control the level of the cleaning and disinfecting solution in the receiving vat (5), a level sensor is provided, which controls the level so that the catheter or catheters (37) situated on the screen insert (7) dip completely into the cleaning and disinfecting solution.

22. A device according to one of Claims 19 to 21,
**characterised in that** the mixture of the cleaning and disinfecting solution takes pace by the amount of mixture removed from the reservoir (111) via a pump (115) being determined by a weighing device (125) determining the weight of the reservoir (111) and being removed until the desired target mixture of water and the mixture is produced in the mixture reservoir (117).

23. A device according to Claim 22,
**characterised in that** the pump (115) conveys at a constant volume flow
and **in that** removal from the reservoir (111) is determined and monitored by recording the operating time of the pump.

24. A device according to Claim 22 or 23,
**characterised in that** the removal from the reservoir (111) is determined or monitored by an electric conductance sensor (139) disposed in a discharge line of the mixture reservoir (117).

25. A device according to one of Claims 19 to 24,
**characterised in that** the cleaning and disinfecting solution can be supplied from the mixture reservoir (117) to the spray nozzle configuration (9) and to the housing part or parts (76) and can be returned from the receiving vat (5) into the mixture reservoir (117).

26. A device according to one of Claims 8 to 25,
**characterised in that** further catheter fixings (41) are provided between adjacent openings (39), so that a catheter (37) laid out over the openings (39) in the housing duct (35) can be fixed in the further catheter fixings (41).

## Revendications

1. Dispositif pour préparer au moins un catheter à ballon (37), qui comporte une pièce de fermeture (44) située à une extrémité proximale et qui par une lumière de ballon (42) est reliée à un ballon (38) disposé dans la zone d'extrémité distale, ainsi qu'une lumière de fil de guidage (40) qui conduit à une sortie de fil de guidage (72) ou à un raccordement propre de fil de guidage (147), dispositif dans lequel :
- il est prévu une cuve à ultrasons (3) contenant un fluide pour produire des ultrasons dans ce fluide, avec une cuve d'accueil (5) immergée dans le fluide et dans laquelle est maintenu un plateau criblant (7) pour accueillir au moins un catheter à ballon,
- au ballon (38) du cathéter à ballon (37) peut être amené, à partir d'une partie de réception (76), par l'intermédiaire de la partie de raccordement de ballon (44) et de la lumière de ballon (42), un mélange composé d'une solution de nettoyage et d'une solution de désinfection, ce mélange pouvant, en appliquant une dépression à la partie de raccordement de ballon (44) ou en exerçant une pression sur le ballon (38), être retiré de celui-ci et de la lumière de ballon (42), le mélange des solutions de nettoyage et de désinfection pouvant être amené à travers la sortie du fil de guidage (72) ou le raccord de fil de guidage (147), à la lumière du fil de guidage (40),
**caractérisé en ce qu'**au-dessus du plateau criblant (7) est prévu un système de buses de pulvérisation auquel peut être amené le mélange des solutions de purification et de désinfection, la cuve à ultrasons (3) pouvant être recouverte avec étanchéité par un couvercle (146) recouvrant le système de buses de pulvérisation (9).

2. Dispositif selon la revendication 1, **caractérisé en ce que** à la cuve à ultrasons (3) peut être amené par une conduite d'amenée (11) le fluide, de l'eau de préférence, et ce fluide peut être retiré de la cuve (3) et renvoyé à la conduite d'amenée (11) par une pompe de recyclage (17).

3. Dispositif selon la revendication 2, **caractérisé en ce qu'**entre la cuve à ultrasons (3) et la pompe de recyclage (17) se trouve une soupape électromagnétique d'évacuation (15), tandis qu'entre la pompe de recyclage (17) et la cuve à ultrasons (3) se trouve une soupape d'amenée (13).

4. Dispositif selon la revendication 2 ou 3, **caractérisé en ce que** dans la cuve à ultrasons (3), il est prévu au moins une sonde à ultrasons (21) plongée dans le fluide..

5. Dispositif selon la revendication 4, **caractérisé en ce qu'**il y a plusieurs sondes à ultrasons (21).

6. Dispositif selon une des revendications 1 à 5, **caractérisé en ce qu'**un dispositif de chauffage (19) est prévu pour amener à une température prédéfinie, le fluide contenu dans la cuve à ultrasons (3).

7. Dispositif selon une des revendications 1 à 6, **caractérisé en ce que** la cuve d'accueil (5) suspendue dans la cuve à ultrasons (3) est fixée en haut sur le bord de la face supérieure de la cuve à ultrasons (3) à l'aide d'un joint d'étanchéité (33), de sorte que la cuve d'accueil (5) ne présente aucun soulèvement dans le fluide de la cuve à ultrasons..

8. Dispositif selon une des revendications 1 à 7, **caractérisé en ce que**
- sur la surface du plateau criblant (7) situé à côté du système de buses de pulvérisation (9) se trouve au moins un canal de réception (35) pour un catheter (37),
- le canal (35) présente dans le plateau criblant (7) plusieurs ouvertures (39) situées l'une derrière l'autre selon la direction longitudinale du canal,
- le canal (35) présente une zone (45) pour le couplage de la lumière de fil de guidage (40) et pour l'amenée du mélange des solutions de nettoyage et de désinfection,
- la partie de réception (76) se trouve d'un côté du canal de réception (35) et peut être reliée de manière étanche à la partie de raccordement de ballon (44) du catheter (37), de manière qu'à la lumière de ballon (42) du catheter (37) peut être amenée la solution de nettoyage et de désinfection et également peut en être retirée.

9. Dispositif selon la revendication 8, **caractérisé en ce que** de l'autre côté du canal de réception (35) est prévue une première ouverture (43) dimensionnée de manière que le ballon (38) du catheter (37), provenant d'un dispositif de fixation (41) situé entre les deux ouvertures (39) suivant la première ouverture (43) passe à travers l'ouverture (39) proche de la première ouverture (43) et sous celle-ci descend obliquement en direction du côté éloigné du système de buses de pulvérisation (9) de sorte qu'il se trouve incliné obliquement vers le bas, ce qui rend possible son dégazage par montée et aspiration des bulles d'air.

10. Dispositif selon la revendication 8 ou 9, **caractérisé en ce que** dans le plateau cribant (7) sont disposés plusieurs canaux de réception parallèles et côte à côte.

11. Dispositif selon une des revendications 8 à 10, **caractérisé en ce que**
- la zone présente un premier joint d'étanchéité élastique (47) disposé en dessous du catheter (37) et fixé sur un support inférieur (49) perpendiculaire au canal,
- dans ce premier joint d'étanchéité (47), se trouve une première zone en creux (73),
- au-dessus du premier joint d'étanchéité (47) et du catheter (37) se trouve un second joint d'étanchéité (53) qui est fixé à une plaque (55) perpendiculaire au canal de réception (37),
- dans le second joint d'étanchéité (53) se trouve une seconde zone en creux (50),
- le second joint d'étanchéité (53) et le premier joint d'étanchéité (47) peuvent être comprimés l'un sur l'autre de sorte que le point de sortie du fil de guidage du catheter (37) est contenu dans un espace étanche constitué par la première et par la seconde zone en creux, espace dans lequel peut être introduit pour rincer la lumière de fil de guidage (40) du catheter (37), le mélange des solutions de nettoyage et de désinfection.

12. Dispositif selon la revendication 11, **caractérisé en ce que** la première zone en creux (73) dans le premier joint d'étanchéité (47) est obtenue en tirant la zone d'étanchéité correspondante, en direction du support inférieur (49) au moyen d'un premier serre-flanc (51).

13. Dispositif selon la revendication 11 ou 12, **caractérisé en ce que** la seconde zone en creux (50) dans le second joint d'étanchéité (53) est obtenue en tirant la zone d'étanchéité correspondante, en direction de la plaque (55) au moyen d'un second serre-flanc (57).

14. Dispositif selon la revendication 13, **caractérisé en ce que** le second serre-flanc (57) comprend une tige (58) traversant le second joint d'étanchéité (53) et qui est percé d'un alésage (59) permettant d'amener le mélange des solutions de nettoyage et de désinfection dans l'espace formé par la première et la seconde zone en creux.

15. Dispositif selon une des revendications 8 à 14, **caractérisé en ce que** le support inférieur (49) et le premier joint d'étanchéité (47) fixé sur lui sont solidaires du plateau criblant (7) en dessous d'un catheter (37), le second joint d'étanchéité (53) peut se déplacer par l'intermédiaire d'un mécanisme (70) perpendiculairement au plan du canal de réception (37) pour ouvrir le premier et le second joint d'étanchéité et pour insérer un catheter (37) ainsi que pour fermer le premier et le second joint d'étanchéité et pour former l'espace.

16. Dispositif selon une des revendications 8 à 15, **caractérisé en ce**
- **que** la partie de réception (76) a la forme d'un bloc (84) qui peut être fixé selon la direction longitudinale du canal de réception (35) en différents points du plateau criblant (7) pour adaptation à des longueurs différentes de catheter (37),
- la partie de raccordement de ballon (44) d'un catheter (37) peut être fixée à un élément de raccordement (93) de la partie de réception (76),
- l'élément de raccordement (93) présente un passage (95) en liaison avec un autre passage (97) prévu dans la partie de réception (76) et qui peut être relié à une conduite d'évacuation (80) pour la solution de nettoyage et de désinfection,
- une conduite d'amenée (78) pour le mélange des solutions de nettoyage et de désinfection est reliée à un autre passage (99) prévu dans la partie de réception et qui est relié au passage (95).

17. Dispositif selon la revendication 16, **caractérisé en ce que** la conduite (78) est reliée à un embout de canule (101) qui pénètre dans le raccord de ballon (44) à travers le passage (95), de sorte que son extrémité libre se trouve un peu en avant de la transition entre la partie de raccord de ballon (44) et la lumière de ballon (42) du catheter (37) et qu'ainsi l'espace mort situé dans la partie de raccord de ballon (44), quand la solution de nettoyage et de désinfection est amenée, est ponté aussi largement que possible.

18. Dispositif selon une des revendications 8 à 17, **caractérisé en ce que** la partie d'accueil (76) présente un autre raccord pour sa liaison avec le raccord de fil de guidage (147) et pour l'amenée du mélange des solutions de nettoyage et de désinfection.

19. Dispositif selon une des revendications 8 à 18, **caractérisé en ce qu'**un mélange constitué d'un agent de nettoyage et d'un agent de désinfection peut être extrait d'un récipient (111) et envoyé à un récipient mélangeur (117) auquel peut être amenée de l'eau par une conduite d'amenée (119), le mélange des solutions de nettoyage et de désinfection issu du récipient mélangeur (117) pouvant être amené au dispositif des buses de pulvérisation (9) ainsi qu'à la partie de réception (76).

20. Dispositif selon la revendication 19, **caractérisé en ce que** le mélange des solutions de nettoyage et de désinfection contenu dans le récipient (117) peut être amené à une température prédéfinie vers un dispositif de chauffage (127) du récipient (117).

21. Dispositif selon une des revendications 1 à 20, **caractérisé en ce que** pour réguler le niveau de la solution de nettoyage et de désinfection dans la cuve de réception (5), il est prévu un capteur de niveau qui régule ce niveau de manière que le ou les catheters (37) se trouvant sur le plateau criblant (7) sont immergés totalement dans la solution de nettoyage et de désinfection.

22. Dispositif selon une des revendications 19 à 21, **caractérisé en ce que** le mélange des solutions de nettoyage et de désinfection s'effectue en mesurant la quantité de mélange prélevée par la pompe (115) dans le récipient (111) au moyen d'un dispositif de pesée (125) déterminant le poids du récipient (111), le prélèvement ayant lieu jusqu'à ce que le mélange désiré composé d'eau et du mélange soit obtenu dans le récipient mélangeur (117).

23. Dispositif selon la revendication 22, **caractérisé en ce que** la pompe (115) a un débit volumétrique constant et que la quantité retirée du récipient (111) est établie et surveillée en captant le temps de fonctionnement de la pompe.

24. Dispositif selon la revendication 22 ou 23, **caractérisé en ce que** la quantité retirée du récipient (111) est établie et surveillée par un capteur de conductance monté dans une conduite de sortie du récipient mélangeur (117).

25. Dispositif selon une des revendications 19 à 24, **caractérisé en ce que** la solution de nettoyage et de désinfection sortant du récipient mélangeur (117) peut être envoyée au système de buses de pulvérisation (9) et aux parties de réception (76), et elle peut être renvoyée au récipient mélangeur (117) à partir de la cuve de réception (5).

26. Dispositif selon une des revendications 8 à 25, **caractérisé en ce qu'**entre deux ouvertures (39) voisines, sont prévues d'autres fixations de catheter (41), de sorte qu'un catheter (37) disposé dans le canal de réception (35) au-dessus des ouvertures (39) peut être fixé dans les autres fixations de catheter (41).
